# EUROPÄISCHE PATENTSCHRIFT

(11) **EP 1 690 536 B1**
(45) Veröffentlichungstag und Bekanntmachung des Hinweises auf die Patenterteilung: **14.05.2008**
(21) Anmeldenummer: 06011207.5
(22) Anmeldetag: 15.11.2000
(51) Int. Cl.: A61K 31/222, A61P 13/06

(54) **Verwendung von r-(+)-2-(3-diisopropylamino-1-phenylpropyl)-4-hydroxy-methyl-Phenylisobuttersäureester Hydrogenfumarat zur Behandlung von Harndrang-Inkontinenz und anderen spasmogenen Leiden**
Use of r-(+)-2-(3-diisopropylamino-1-phenylpropyl)-4-hydroxy-methyl-phenylisobutyric acid ester hydrogenfumarate for the treatment of urinary incontinence and other spasmogenic disorders
Utilisation de l' hydrogène fumarate de l'ester de l'acide r-(+)-2-(3-diisopropylamino-1-phenylpropyl)- 4-hydroxy-methyl-phenylisobutyrique pour le traitement de l'incontinence urinaire et d'autres maladies spasmogeniques

(30) Priorität: 16.11.1999 DE 19955190
(43) Veröffentlichungstag der Anmeldung: 16.08.2006
(62) Teilanmeldung aus: 04018487.1
(73) Patentinhaber: SCHWARZ PHARMA AG, 40789 Monheim (DE)
(72) Erfinder: Meese, Claus, 40789 Monheim (DE)
(74) Vertreter: HOFFMANN EITLE

(56) Entgegenhaltungen:
- EP-A- 0 957 073
- WO-A-99/58478
- PALMER L ET AL: "DETERMINATION OF TOLTERODINE AND THE 5-HYDROXYMETHYL METABOLITE IN PLASMA, SERUM AND URINE USING GAS CHROMATOGRAPHY-MASS SPECTROMETRY" JOURNAL OF PHARMACEUTICAL AND BIOMEDICAL ANALYSIS, NEW YORK, NY, US, Bd. 16, Nr. 1, 1997, Seiten 155-165, XP000995770 ISSN: 0731-7085

## Beschreibung

Die vorliegende Erfindung betrifft die Verwendung von *R*-(+)-2-(3-Diisopropylamino-1-phenylpropyl)-4-hydroxy-methyl-phenylisobuttersäureester Hydrogenfumarat zur Herstellung eines Medikaments zur Behandlung von Harndrang-Inkontinenz und anderen spasmogenen Leiden und pharmazeutische Formulierungen umfassend *R*-(+)-2-(3-Diisopropylamino-1-phenylpropyl)-4-hydroxy-methyl-phenylisobuttersäureester Hydrogenfumarat.

Aus dem Dokument PCT/EP99/03212 sind neuartige Derivate von 3,3-Diphenylpropylaminen bekannt.

Sie sind wertvolle Prodrugs für die Behandlung von Harndrang-Inkontinenz und anderen spasmogenen Leiden, die den Nachteil bisher zur Verfügung stehender Wirkstoffe, nämlich zu geringe Adsorption der Wirkstoffe durch biologische Membranen oder deren ungünstigen Metabolismus vermeiden.

Weiterhin zeichnen sich diese neuartigen Prodrugs durch verbesserte pharmakokinetische Eigenschaften im Vergleich zu Oxybutynin und Tolterodin aus.

Bevorzugte Verbindungen aus der Gruppe dieser neuartigen Derivate von 3,3-Diphenylpropylaminen sind Ester aliphatischer oder aromatischer Carbonsäuren mit der nachfolgend genannten allgemeinen Formel A 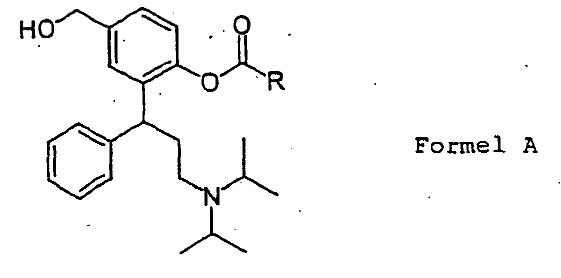 in der R die Bedeutung von C₁-C₆-alkyl, C₃-C₁₀-cycloalkyl oder unsubstituiertem oder substituiertem Phenyl hat. Sie können in Form ihrer optischen Isomere, als Racematengemisch und in Form ihrer individuellen Enantiomere vorliegen.

Verbindungen der Struktur der Formel A besitzen allerdings eine geringe Wasserlöslichkeit. Diese verringert ihre orale Bioverfügbarkeit.

Schließlich neigen Monoester der Struktur, wie sie in Formel A wiedergegeben sind, zu intermolekularer Umesterung.

Bei längerer Lagerung ist deshalb unter Gehaltsabnahme von Verbindungen der Struktur der allgemeinen Formel A eine Zunahme von Diester und freiem Diol feststellbar.

Zwar lassen sich grundsätzlich Salze der Verbindungen der allgemeinen Formel A erhalten, indem Lösungen der Verbindungen der Formel A (Basenteil) mit Lösungen von Säuren in jeweils geeigneten Lösungsmitteln vereinigt werden, jedoch erweisen sich die als Festkörper erhaltenen Salze als durchweg amorph und/oder hygroskopisch und sind auch aus den üblichen Lösungsmitteln nicht ohne weiteres kristallisierbar. Derartige Salze weisen eine zu geringe chemische Stabilität auf, um als wertvolle pharmazeutische Wirkstoffe galenisch verarbeitet werden zu können.

Es ist daher Aufgabe der vorliegenden Erfindung, eine Verbindung eines hochreinen, kristallinen, stabilen Derivates von 3,3-Diphenylpropylaminen in Form eines Salzes zur Verfügung zu stellen, das die erwähnten Nachteile vermeidet und sich besonders gut zum Einsatz in pharmazeutisch-technischen Formulierungen eignet und zu solchen verarbeiten lässt.

Diese Aufgabe wurde durch den Gegenstand der Patentansprüche gelöst.

Die *R*-konfigurierte Verbindung der Formel 2 wird nach dem folgenden Verfahren hergestellt 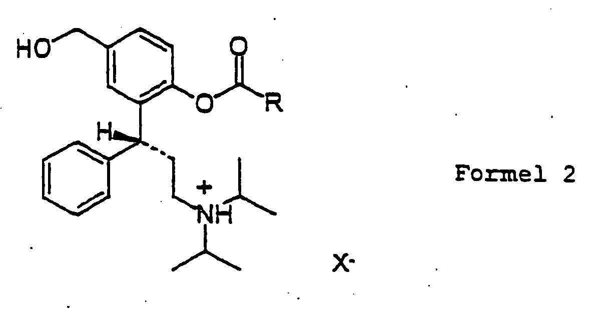 worin R für Isopropyl steht und X⁻ für den Säurerest Hydrogenfumarat steht, indem
a) eine Verbindung der Formel 3 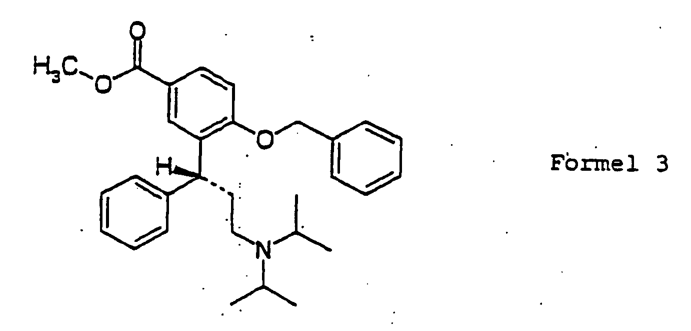 mit einem Hydrierungsmittel zur Bildung einer Verbindung der Formel 5 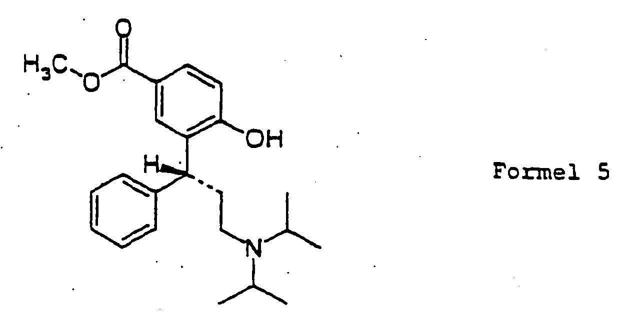 gespalten wird, worauf
b) die so erhaltene Verbindung der Formel 5 mit einem Reduktionsmittel umgesetzt wird, um eine Verbindung der Formel 6 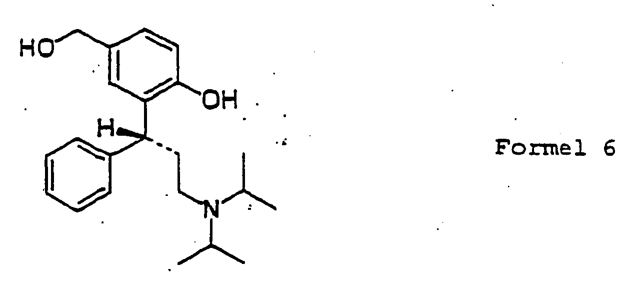 zu ergeben, die
c) mit einem Acylierungsmittel umgesetzt wird, um eine Verbidung der Formel 1 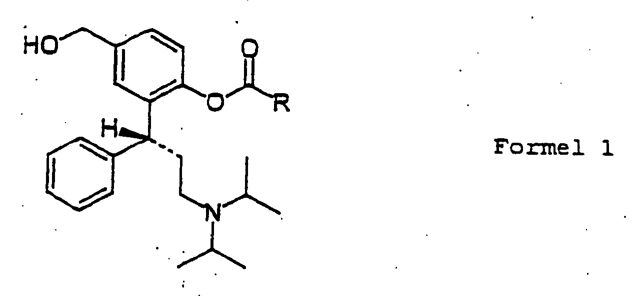 zu erhalten, worin R die oben genannte Bedeutung hat, die
d) mit Fumarsäure unter der Bildung einer Verbindung der Formel 2 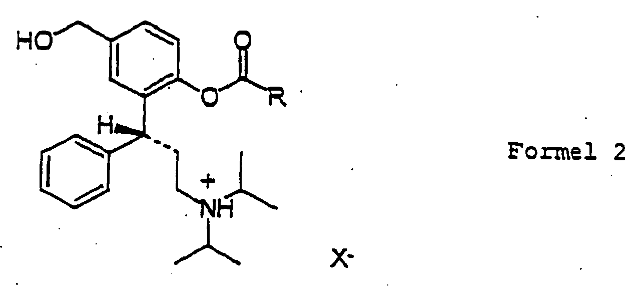 umgesetzt wird, worin R für Isopropyl steht und X⁻ für den Säurerest Hydrogenfumarat steht.

Besonders vorteilhaft wird, ausgehend von dem kristallinen *R-*(-)-4-Benzyloxy-3-(3-diisopropylamino-1-phenyl-propyl)benzoesäuremethylester, das hochreine, kristalline Zwischenprodukt *R*-(-)-3-(3-Diisopropylamino-phenyl-propyl)-9-hydroxy-benzoesäuremethylester dargestellt, das zu *R*-(+)-2-(3-Diisopropylamino-1-phenylpropyl)-4-hydroxymethylphenol reduziert wird, schließlich geeignet acyliert wird und anschließend mit Fumarsäure unter spontaner Kristallisation zum hochreinen, kristallinen, stabilen Salz umgesetzt wird.

Es wird die Verbindung der Formel 1 erhalten; 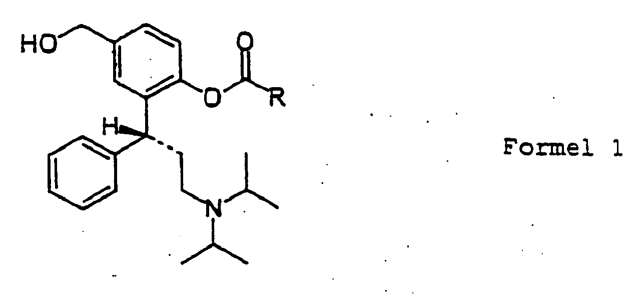 in der R die Bedeutung von Isopropyl hat.

Zum Erhalt der Verbindung in Form ihres Salzes ist die spezielle Reaktionsführung über besondere Zwischenstufen und individualisierbare Zwischenprodukte entscheidend.

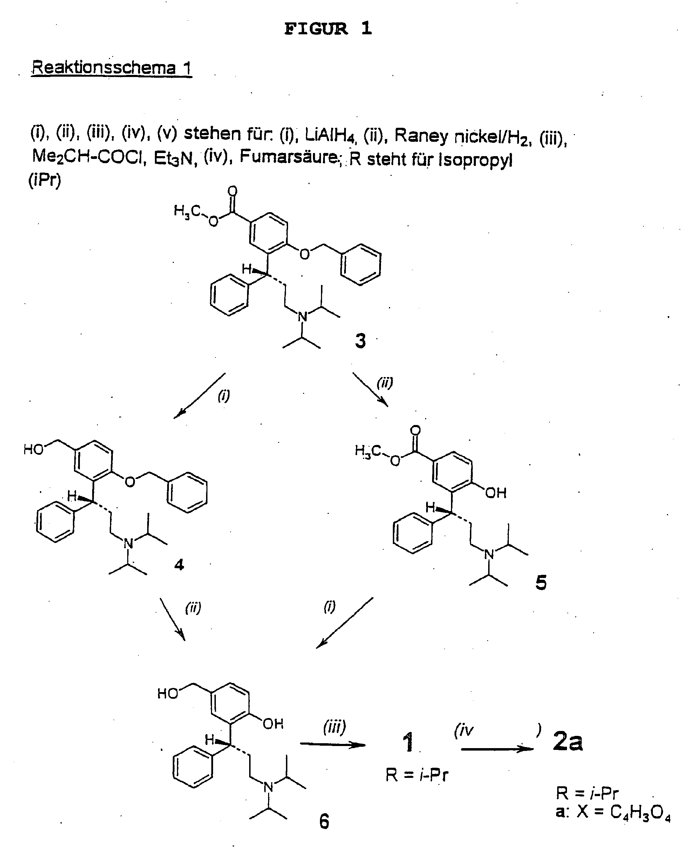

Dies Wird anhand des Reaktionsschemas 1 (siehe Figur 1) erläutert, in dem Umsetzungen mit *R*-konfigurierten Verbindungen, beschrieben werden.

Darin bedeuten:

| | |
|---|---|
| 3 | = *R*-(-)-4-Benzyloxy-3-(3-diisoprapylamino-1-phenyl-propyl)-benzoesäure- methylester |
| 4 | = *R*-(+)-[4-Benzyloxy-3-(3-diisopropylamino-1-phenyl-propyl)-phenyl]-methanol |
| 5 | = *R*-(-)-3-(3-Disopropylamino-phenyl-propyl)-4-hydroxy-benzoesäuremethylester |
| 6 | = *R*-(+)-2-(3-Diisopropylamino-1-phenylpropyl)-4-hydroxymethylphenol |
| 1 | = *R*-(+)-2-(3-Diisopiopylamino-1-phenylpropyl)-4-hydroxymethylphenyl-isobuttersäureester |
| 2a | = *R*-(+)-2-(3-Diisopzopylamino-1-phenylpropyl)-4-hydroxymethylphenyl-isobuttersäureester Hydrogenfumarat |

Entsprechend der in den Ausführungsbeispielen erläuterten Reaktionsführung wird die Vorstufe 3 (*R*-(-)-4-Benzyloxy-3-(3-diisopropylamino-1-phenyl-propyl)-benzoesäure-methylester) kristallin und rein dargestellt.

Vorstufe 3 wird nach üblichen Methoden - z.B. BBr₃, AlCl₃ - vorzugsweise jedoch mittels Wasserstoffgas über Raney-Nickel in Methanol als Lösungsmittel bei Raumtemperatur (RT) zu 5 (*R*-(-)-3-(3-Diisopropylamino-phenyl-propyl)-4-hydroxy-benzoesäuremethylester) gespalten. Dieses fällt in hochreiner, kristalliner Form (Schmp. 143.7 °C) an.

Schließlich wird 5 mit einem geeigneten Reduktionsmittel - z.B. NaBH₄/EtOH - vorzugsweise LiAlH₄ in einem inerten Lösungsmittel bei niedrigen Temperaturen (-78°C bis + 10°C) reduziert und die Verbindung 6 (*R*-(+)-2-(3-Diisopropylamino-Diethylether gelöst, zweimal mit je 100 ml Wasser gewaschen, über Natriumsulfat getrocknet, filtriert und zur Trockene eingeengt. Man erhält 14.1 g (90.4 % der Theorie) *R*-(+)-2-(3-Diisopropylamino-1-phenylpropyl)-4-hydroxymethylphenol in Form eines cremefarbenen, amorphen Festkörpers. Umkristallisation siehe unten c).

### b) Ausgehend von der Zwischenstufe (5); R-(-)-3-(3-Diisopropylamino-phenyl-propyl)-4-hydroxy-benzoesäuremethylester

Eine Lösung von 370 mg (1.0 mmol) *R*-(-)-3-(3-Diisopropylamino-phenyl-propyl)-4-hydroxy-benzoesäuremethylester in 20 ml wasserfreiem Tetrahydrofuran wird langsam und bei Raumtemperatur zu einer gerührten Mischung von trockenem Tetrahydrofuran (10 ml) und einer 1M Lösung von Lithiumaluminiumhydrid in Tetrahydrofuran (3 ml) (unter Stickstoffschutzgasatmosphäre) getropft. Überschüssiges Hydrid wird durch tropfenweisen Zusatz einer gesättigten Natriumcarbonatlösung zersetzt. Nach dem Abtrennen der organischen Phase wird diese am Rotationsverdampfer eingeengt und anschließend im Hochvakuum getrocknet. Es werden 274 mg (74 % der Theorie) blaßgelbes Öl erhalten, das sich langsam zu einer amorphen Masse verfestigt.

### c) Umkristallisation:

Rohprodukt 6 (1.0 g) wird in Ethylacetat gelöst und am Rotationsverdampfer erneut eingeengt. Das so von Fremdlösemitteln (Diethylether bzw. Tetrahydrofuran, s.o.) befreite Diol wird unter leichtem Erwärmen mit 1.5 ml Ethylacetat versetzt. Man rührt, bis eine klare Lösung entstanden ist, läßt auf Raumtemperatur abkühlen und setzt einige Impfkristalle zu. Letztere werden gewonnen, indem rohes 6 über HPLC gereinigt wird, die Hauptfraktion aufgefangen wird, eingeengt und der Rückstand mehrere Stunden im Hochvakuum getrocknet wird. Nachdem deutliche Kristallisation eingetreten ist, beläßt man bei - 10 °C. Die Kristalle werden noch in der Kälte abgesaugt und im Vakuum getrocknet. Man erhält farblose Kristalle in 84 % Ausbeute.
Schmp. 102.3 °C
DC (1) : 0.57
[I]_{D}²⁰ = + 21.3 (c = 1.0, Ethanol).
¹³C-NMR (CDCl₃): 19.58, 19.96, 33.30, 39.52, 42.10, 48.00, 65.40, 118.58, 126.31, 126.57, 127.16, 127.54, 128.57, 132.63, 132.83, 144.55, 155.52.

### 5. Darstellung von R-(+)-2-(3-Diisopropylamino-1-phenylpropyl)-4-hydroxymethylphenylisobuttersäureester (1)

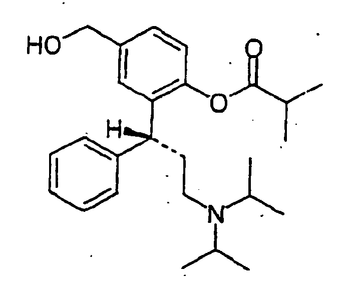

Eine Lösung von *R*-(+)-2-(3-Diisopropylamino-1-phenylpropyl)-4-hydroxymethylphenol (6) (65.0 g, 190.3 mmol) und Triethylamin (20.4 g, 201.7 mmol) in 750 ml Dichlormethan wird unter Rühren und Kühlung (-5 °C) mit einer Lösung von Isobuttersäurechlorid (23.4 g, 201.7 mmol) in 250 ml Dichlormethan vernetzt Nach der Zugabe wird noch 15 Min. bei 0 °C, dann 30 Min. bei Raumtemperatur gerührt und nacheinander mit Wasser (250 ml) und 5%-iger wässriger Natriumhydrogencarbonat-Lösung gewaschen. Die organische Phase wird abgetrennt und am Rotationsverdampfer zur Trockene eingeengt. Der Ester *R*-(+)-2-(3-Diisopropylamino-1-phenylpropyl)-4-hydroxymethylphenylisobuttersäureester wird als farbloses, viskoses Öl erhalten; Ausbeute: 77.1 g (98.4 % der Theorie).
DC (1) : 0.26; [I]_{D}²² = + 2.7 (c = 1.0, Ethanol).
¹³C-NMR (CDCl₃):19.01, 19. 95, 20. 59, 21.12, 34.28, 36.89, 41.88, 42.32, 43.90, 48.78, 64.68, 122.57, 125.59, 126.16, 126.86, 127.96, 128.54, 136.88, 138.82, 143.92, 147.90, 175.96.

### 6. Darstellung von R-(+)-2-(3-Diisopropylamino-1-phenylpropyl)-4-hydroxymethylphenylisobuttersäureester Hydrogenfumarat

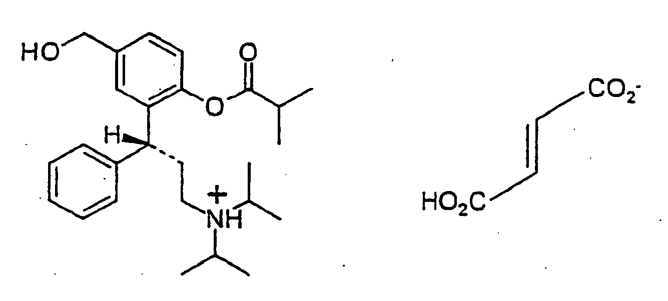

Eine Lösung von 41.87 g (102 mmol) *R*-(+)-2-(3-Diisopropylamino-1-phenylpropyl)-4-hydroxymethylphenylisobuttersäureester in 90 ml 2-Butanon wird unter Erwärmen mit Fumarsäure (11.81 g, 102 mmol) versetzt. Nach dem Lösen der Säure wird langsam unter Rühren Cyclohexan (20-30 ml) bis zum Einsetzen einer Trübung zugesetzt. Man beläßt den farblosen, homogenen Ansatz zunächst 18 Stunden bei Raumtemperatur, dann mehrere Stunden bei 0 °C. Die ausgefallenen farblosen Kristalle werden abgesaugt, mit wenig Cyclohexan/2-Butanon (90:10, Vol.-%) gewaschen und im Vakuum bei 30 °C getrocknet. Man erhält 44.6 g (83.1 % der Theorie) des Hydrogenfumarat-Salzes des *R*-(+)-2-(3-Diisopropylamino-1-phenylpropyl)-4-hydroxymethylphenylisobuttersäureester in Form farbloser Plättchen.

Schmp. 98.8 °C, eine zweite Kristallisation aus dem gleichen Lösungsmittelgemisch ergibt das Produkt mit einem Schmp. von 103 °C.
[I]_{D}²⁰ = + 6.0 (c = 1.0, Ethanol).
Elementaranalyse: Berechnet für C₃₀H₄₁NO₇ (Molgewicht 527.66) C 68.29 %, H 7.83 %, N 2.65 %, O 21.2 %; gefunden C 68.29 %, H 7.90 %, N 2.72 %, O 21.0 %.
UV/VIS bei Σ in nm (A ^{1 %} _{1 cm}): 191 (1306), 193 (1305), 200 (1143), 220 (456).
IR: 3380, 2978, 2939, 2878, 2692, 2514, 1756, 1702, 1680, 1618, 1496, 1468, 1226, 1040, 1019, 806,
¹H-NMR (CDCl₃) : 1.198, 1.285, 1.287 (CH₃) ; 2.541 (CHC=O) ; 3.589 (NCH); 4.585 (CH₂OH); 6.832 (=CH, Fumarat); 6.84-7.62 (Aryl, = CH).
¹³C-NMR (CDCl₃): 17.79, 18.95, 19.16 (CH₃); 31.63 (CHCH₂) ; 34.09 (CH-C=O); 41.87 (CHCH₂); 45.83 (NCH₂); 54.29 (NCH); 63.78 (OCH₂); 122.23, 126.48, 126.77, 127.56, 140.46, 140.52, 142.35, 147.54 (Aryl CH); 135.54 (=CH, Fumarat); 170.48 (C=O, Fumarat); 175.62 (i-Pr-C=O).

MS im Direkteinlaß, m/z (%): 411 (1), 396 (9), 380 (1), 223 (2), 165 (2), 114 (100), 98 (4), 91 (3), 84 (3), 72 (10), 56 (7).

## Patentansprüche

1. Verwendung von *R*-(+)-2-(3-Diisopropylamino-1-phenylpropyl)-4-hydroxy-methyl-phenylisobuttersäureester Hydrogenfumarat zur Herstellung eines Medikaments zur Behandlung von Harndrang-Inkontinenz und anderen spasmogenen Leiden.

2. Verwendung nach Anspruch 1, wobei das *R*-(+)-2-(3-Diisopropylamino-1-phenylpropyl)-4-hydroxy-methyl-phenylisobuttersäureester Hydrogenfumarat bei der Herstellung besagten Medikaments in kristalliner Form verwendet wird.

3. Verwendung nach einem der Ansprüche 1 oder 2, wobei das Medikament zur Behandlung von Harndrang-Inkontinenz verwendbar ist.

4. Pharmazeutische Formulierung umfassend *R*-(+)-2-(3-Diisopropylamino-1-phenylpropyl)-4-hydroxy-methyl-phenylisobuttersäureester Hydrogenfumarat.

5. Pharmazeutische Formulierung nach Anspruch 3, herstellbar durch die Verwendung von *R*-(+)-2-(3-Diisopropylamino-1-phenylpropyl)-4-hydroxy-methyl-phenylisobuttersäureester Hydrogenfumarat in kristalliner Form.

6. R-(+)-2-(3-Diisopropylamino-1-phenylpropyl)-4-hydroxymethyl-phenylisobuttersäureester Hydrogenfumarat zur Verwendung in einem Verfahren zur Behandlung von Harndranginkontinenz.

## Claims

1. Use of R-(+)-2-(3-diisopropylamino-1-phenylpropyl)-4-hydroxy-methyl-phenylisobutyrate hydrogenfumarate for the preparation of a medicament for the treatment of urinary incontinence and other spasmogenic diseases.

2. Use according to claim 1, wherein R-(+)-2-(3-diisopropylamino-1-phenylpropyl)-4-hydroxy-methyl-phenylisobutyrate hydrogenfumarate is used in crystalline form in the preparation of said medicament.

3. Use according to any of claims 1 or 2, wherein the medicament is useable for the treatment of urinary incontinence.

4. Pharmaceutical formulation comprising R-(+)-2-(3-diisopropylamino-1-phenylpropyl)-4-hydroxy-methyl-phenylisobutyrate hydrogenfumarate.

5. Pharmaceutical formulation according to claim 4, producible by using R-(+)-2-(3-diisopropylamino-1-phenylpropyl)-4-hydroxy-methyl-phenylisobutyrate hydrogenfumarate in crystalline form.

6. R-(+)-2-(3-diisopropylamino-1-phenylpropyl)-4-hydroxy-methyl-phenylisobutyrate hydrogenfumarate for use in a method for the treatment of urinary incontinence.

## Revendications

1. Utilisation de l'hydrogénofumarate de l'ester isobutyrique de R-(+)-2-(3-di-isopropylamino-1-phénylpropyl)-4-hydroxyméthyl-phényle pour la fabrication d'un médicament destiné au traitement de l'incontinence urinaire d'urgence et d'autres troubles spasmodiques.

2. Utilisation selon la revendication 1, dans laquelle l'hydrogénofumarate de l'ester isobutyrique de R-(+)-2-(3-di-isopropylamino-1-phénylpropyl)-4-hydroxyméthyl-phényle est utilisé sous forme cristalline pour fabriquer ledit médicament.

3. Utilisation selon l'une quelconque des revendications 1 ou 2, dans laquelle le médicament est utilisable pour le traitement de l'incontinence urinaire d'urgence.

4. Formulation pharmaceutique comprenant de l'hydrogénofumarate de l'ester isobutyrique de R-(+)-2-(3-di-isopropylamino-1-phénylpropyl)-4-hydroxyméthyl-phényle.

5. Formulation pharmaceutique selon la revendication 4, pouvant être fabriquée par l'utilisation d'hydrogénofumarate de l'ester isobutyrique de R-(+)-2-(3-di-isopropylamino-1-phénylpropyl)-4-hydroxyméthyl-phényle sous forme cristalline.

6. Hydrogénofumarate de l'ester isobutyrique de R-(+)-2-(3-di-isopropylamino-1-phénylpropyl)-4-hydroxyméthyl-phényle, destiné à être utilisé dans un procédé pour le traitement de l'incontinence urinaire d'urgence.
